# EUROPEAN PATENT APPLICATION

(11) **EP 4 257 706 A1**
(43) Date of publication of application: **11.10.2023**
(21) Application number: 22183070.6
(22) Date of filing: 05.07.2022
(51) Int. Cl.: C12Q 1/70, G01N 1/40, G01N 33/569

(54) **SPECIMEN COLLECTION SOLUTION FOR ONE-STEP RAPID ANTIGEN DIAGNOSTIC TEST**

(30) Priority: 08.04.2022 KR 20220043909
(71) Applicant: BIO3S Co., Ltd., Gwangju 61186 (KR)
(72) Inventor: CHO, Jong Youn, 22736 SEO-GU, INCHEON (KR)
(74) Representative: ZBM Patents - Zea, Barlocci & Markvardsen

(57) **Abstract**

The present disclosure relates to a specimen collection solution for a one-step rapid antigen diagnostic test. More specifically, the present disclosure relates to a specimen collection solution including soybean-derived terminally truncated canavalin, sodium chloride, a surfactant, and an emulsifier. Moreover, the present disclosure relates to a diagnostic kit and diagnostic method for a one-step rapid antigen diagnostic test including the specimen collection solution.

## Description

### CROSS-REFERENCE TO RELATED APPLICATION

This application claims the benefit of Korean Patent Application No. 10-2022-0043909 filed on April 8, 2022, in the Korean Intellectual Property Office, the entire disclosure of which is incorporated herein by reference for all purposes.

### BACKGROUND

### 1. Field of the Invention

One or more example embodiments relate to a specimen collection solution for a one-step rapid antigen diagnostic test and its utilization. For example, the utilization relates to a diagnostic kit for a one-step rapid antigen diagnostic test and a one-step rapid antigen diagnostic test method.

### 2. Description of the Related Art

The novel coronavirus (COVID-19), which first found in 2019, has spread around the world, causing serious social and economic issues. Due to the development of trade between countries and the increase in travel, the spread of the virus has progressed rapidly, and the World Health Organization (WHO) declared a pandemic on March 11, 2020. The worldwide spread of infectious diseases caused by viruses occurs in various forms, such as avian influenza in 2008 and foot-and-mouth disease in 2010, in addition to SARS (severe acute respiratory syndrome) in 2003, swine flu in 2009, and MERS (Middle East respiratory syndrome) in 2015.

Currently, the world standard test method for COVID-19 set by the WHO is a PCR test. Currently, professional medical staff collects samples from noses of patients and conducts PCR testing. RT-PCR testing method shows high sensitivity and is convenient for examining samples from many patients. However, there are disadvantages in that infection of a medical staff is expected in the process of collecting a sample, professional medical staff and special equipment are required to proceed with the test and data analysis, and the cost is high, so the test may not be conducted on a large population.

There is an increasing need for technology that allows a sample to be collected as easily as a patient's saliva and performs a rapid/accurate detection by desorbing a virus that is strongly attached to the oral cavity or nasal cavity. In addition, a rapid test kit that is convenient, portable, and capable of self-diagnosis is being actively developed.

A rapid kit product for rapid *in vitro* diagnosis enables on-site diagnosis using visual identification and has the advantage of being able to diagnose quickly and easily without large-scale analysis equipment. Various types of rapid kits are being developed, but the limitation of rapid kits is low sensitivity, which may lead to a reluctant use thereof. The saliva of a patient has a very high viscosity, so it is difficult to apply the same directly to a diagnostic kit. Collecting a sample by a RT-PCR (reverse transcription polymerase chain reaction) method is performed by medical staff, and there is a limitation in self-diagnosis because it is inconvenient for a testee to collect a specimen by himself/herself.

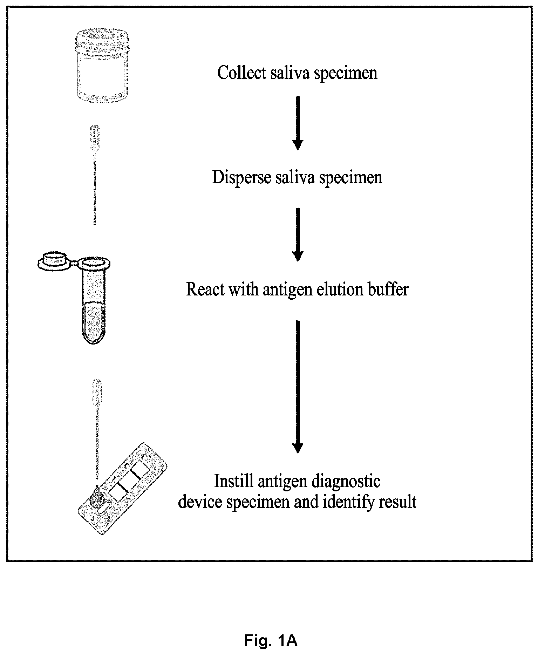

Moreover, as shown in FIG. 1A, commonly known *in vitro* diagnostic devices (In Vitro Diagnostics, IVD) use a specimen collection tool (swab) or collect saliva in a specimen container and then react with a buffer for antigen extraction. Hence, in order to secure the sensitivity and speed of diagnosis using an *in vitro* diagnostic device, it is necessary to simplify the specimen collection and pretreatment process prior to the instillation of a specimen.

### SUMMARY

An aspect of the present disclosure is directed to providing a specimen collection solution for a rapid antigen diagnostic test, which may improve the performance (for example, sensitivity) of the rapid antigen diagnostic test while minimizing specimen pretreatment.

An aspect of the present disclosure is directed to providing a diagnostic kit for a rapid antigen diagnostic test that minimizes the stages of an antigen diagnostic test, improves performance (for example, sensitivity), and improves test convenience using the specimen collection solution according to the present disclosure.

An aspect of the present disclosure is directed to providing a rapid antigen diagnostic test method (for example, a one-step rapid antigen diagnostic test method) using the specimen collection solution according to the present disclosure.

However, the aspects of the present disclosure are not limited to those mentioned above, and other aspects not mentioned herein will be clearly understood by those skilled in the art from the following description.

According to an example embodiment of the present disclosure, there is provided a specimen collection solution including soybean-derived terminally truncated canavalin, sodium chloride, a surfactant, and an emulsifier.

According to an example embodiment of the present disclosure, the specimen collection solution may include 0.1 wt% to 40 wt% of soybean-derived terminally truncated canavalin, 0.1% wt% to 5 wt% of sodium chloride, 0.01 wt% to 2 wt% of a surfactant, and 0.01 wt% to 2 wt% of an emulsifier.

According to an example embodiment of the present disclosure, the soybean-derived terminally truncated canavalin may be C-terminally truncated canavalin.

According to an example embodiment of the present disclosure, the soybean-derived terminally truncated canavalin may include a C-terminally truncated sequence among canavalin amino acid sequences, and the terminally truncated canavalin may be derived from Canavalia gladiata or a fermented soybean.

According to an example embodiment of the present disclosure, the soybean-derived terminally truncated canavalin may be extracted from a fermentation product obtained by inoculating Bacillus subtilus into soybeans, soybean hulls or both and then fermenting the same at 30°C to 40°C.

According to an example embodiment of the present disclosure, the surfactant may include an anionic surfactant, a cationic surfactant, or both, and the surfactant may include at least one of sodium lauryl sulfate, sodium myristyl sulfate, sodium N-lauroyl sarcosinate, sodium N-myristyl sarcosinate, monoglyceride sulfate, sodium lauroyl sarcosinate, lauric acid diethanolamide, polyoxyethylene, lauric acid diethanolamide, N-lauryldiamino ethyl glycine, N-myristyl diamino ethyl glycine, N-alkyl-N-carboxymethyl ammonium betaine, and sodium 2-alkyl-1-hydroxyethyl imidazoline betaine.

According to an example embodiment of the present disclosure, the emulsifier may include at least one of polysorbate 80, polysorbate 60, polysorbate 20, sucrose fatty acid ester, glycerin fatty acid ester, propylene glycol fatty acid ester, sorbitan fatty acid ester, organic acid monoglyceride, and lecithin.

According to an example embodiment of the present disclosure, a mixing ratio of the surfactant to the emulsifier may be in a range of 0.01 : 1 to 1.5 : 1 (w/w).

According to an example embodiment of the present disclosure, the specimen collection may be collected from an oral cavity or nasal cavity.

According to an example embodiment of the present disclosure, the solution may be a gargle solution.

According to an example embodiment of the present disclosure, there is provided a diagnostic kit for a one-step rapid antigen diagnostic test, in which the diagnostic kit includes the specimen collection solution and a rapid antigen diagnostic device according to the present disclosure.

According to an example embodiment of the present disclosure, the diagnostic kit may be for diagnosing respiratory viruses.

According to an example embodiment of the present disclosure, the diagnostic kit may be for diagnosing COVID-19.

According to an example embodiment of the present disclosure, the diagnostic kit may be configured to directly inject a specimen solution collected as the specimen collection solution into a specimen instillation site of a rapid antigen diagnostic device and perform a test.

According to an example embodiment of the present disclosure, the diagnostic kit may be free of a buffer solution for antigen extraction.

According to an example embodiment of the present disclosure, there is a provided a method for a one-step rapid antigen diagnostic test, in which the method includes collecting a specimen solution as the specimen collection solution according to the present disclosure, and directly injecting the specimen solution into a rapid antigen diagnostic device and performing a test.

According to an example embodiment of the present disclosure, the collecting of the specimen may include performing gargling in the oral cavity.

According to an example embodiment of the present disclosure, the performing of the gargling may include performing gargling for 10 seconds to 10 minutes.

According to an example embodiment of the present disclosure, the specimen solution in the injecting of the specimen solution into the rapid antigen diagnostic device and the performing of the test may be free of a buffer solution for antigen extraction.

According to an example embodiment of the present disclosure, an aspect of the present disclosure provides a specimen collection solution, which can improve the performance (for example, sensitivity) of a rapid antigen diagnostic test while minimizing specimen pretreatment and can be utilized for a rapid antigen diagnostic test.

According to an example embodiment of the present disclosure, the specimen collection solution according to the present disclosure may increase the desorption of viruses attached to tissues (for example, utilized as a solution for oral or nasal specimen collection), substrate, etc. for rapid antigen testing to facilitate virus collection and improve the performance of rapid antigen diagnostic tests (for example, sensitivity).

According to an example embodiment of the present disclosure, an aspect of the present disclosure provides a diagnostic kit for a one-step rapid antigen diagnostic test and a diagnostic method using the same capable of minimizing the stages of a rapid antigen diagnostic test, improving test performance (for example, sensitivity), and improving test convenience for users by utilizing a specimen collection solution.

According to an example embodiment of the present disclosure, the specimen collection solution according to the present disclosure may be utilized as a solution for oral saliva specimen collection, which can realize a one-step rapid antigen test. Moreover, it is possible to collect a specimen for diagnosis from within the human body (for example, in the oral cavity) by a simple and convenient method such as gargling and directly introduce the same into a diagnostic device, thereby minimizing the stages of a rapid antigen diagnostic test and improving sensitivity.

### BRIEF DESCRIPTION OF THE DRAWINGS

These and/or other aspects, features, and advantages of the invention will become apparent and more readily appreciated from the following description of example embodiments, taken in conjunction with the accompanying drawings of which:
FIG. 1A is a schematic diagram of a process flow of a rapid antigen diagnostic test method according to a related art;

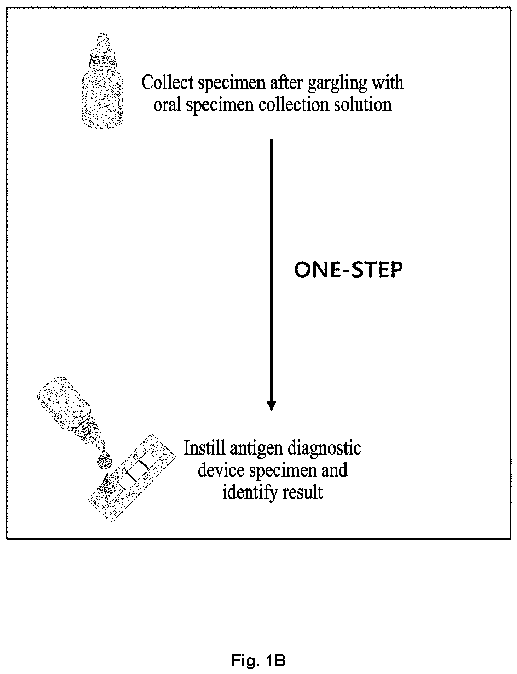

FIG. 1B is an exemplary schematic diagram of a process flow of a one-step rapid antigen diagnostic test method according to an example embodiment of the present disclosure;

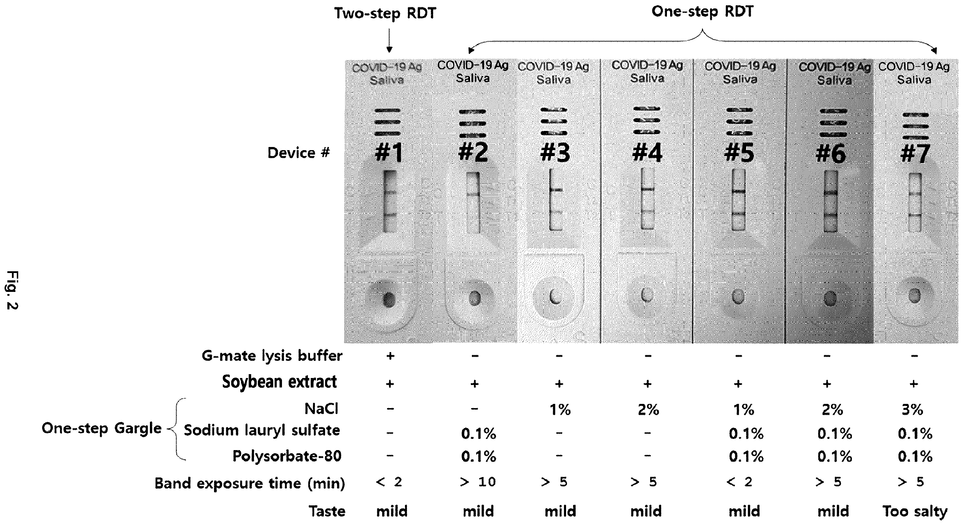

FIG. 2 illustrates an evaluation of the performance results of a COVID-19 antigen diagnostic test (RDT) performed using an oral specimen collection solution according to an example embodiment of the present disclosure in examples of the present disclosure, according to an example embodiment of the present disclosure;

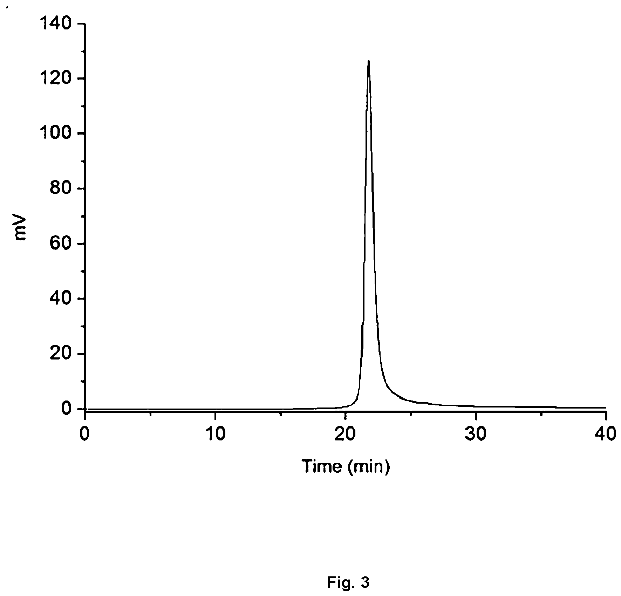

FIG. 3 is a schematic diagram illustrating a chromatogram obtained by purifying the fraction protein obtained by fermenting Canavalia gladiate using Size Exclusion Chromatography in examples of the present disclosure, according to an example embodiment of the present disclosure;

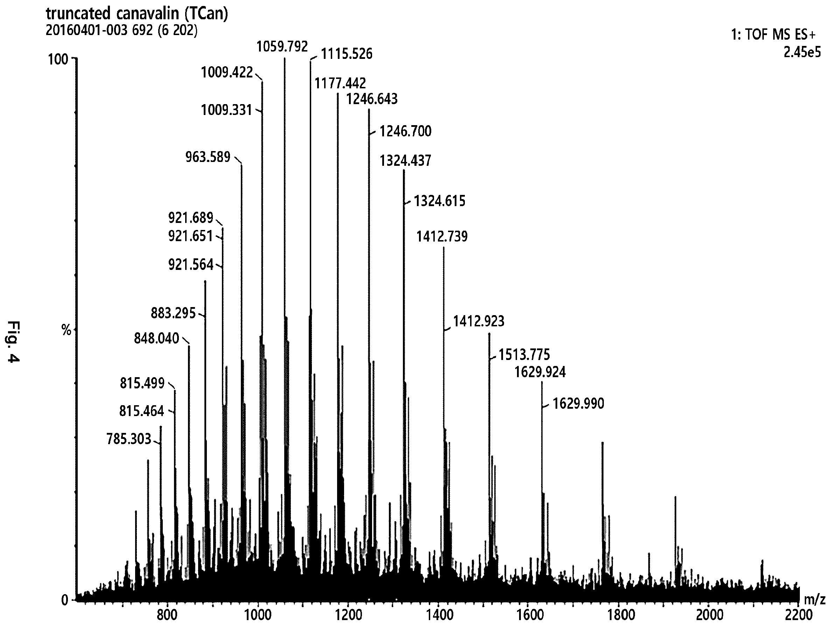

FIG. 4 illustrates basic data for calculating a mass value by obtaining an intact mass value using a mass spectrometer for a TCan protein in examples of the present disclosure, according to an example embodiment of the present disclosure;

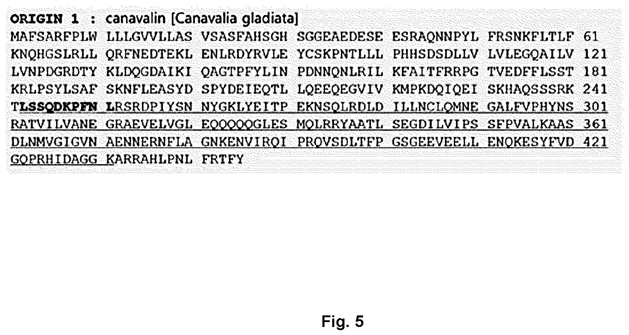

FIG. 5 illustrates a truncation portion from the entire protein sequence obtaining an LSSQDKPFNL sequence from an N-terminal sequencing method from the entire protein sequence of canavalin obtained in examples of the present disclosure (boldface of LSSQDKPFNL portion), and illustrates a sequence portion of the entire protein corresponding to the mass value calculated utilizing the protein mass value information of FIG. 4 (underlined portion), according to an example embodiment of the present disclosure;

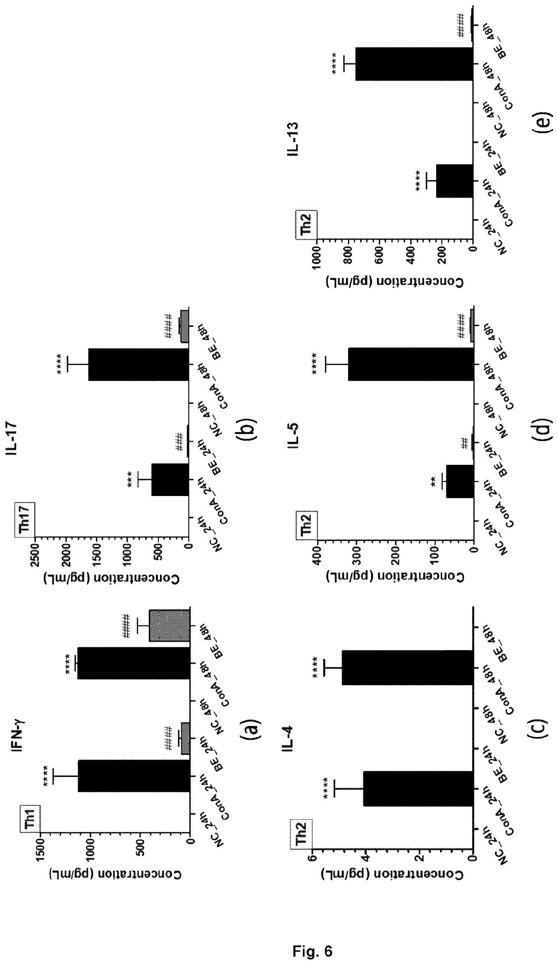

FIGS. 6A to 6E illustrate examples of a change in an expression level of inflammatory cytokines in examples of the present disclosure, according to an example embodiment of the present disclosure, FIG. 6A illustrates a change in an expression level of interferon gamma (IFN-g), FIG. 6B illustrates a change in an expression level of interleukin (IL)-17, FIG. 6C illustrates a change in an expression level of IL-4, FIG. 6D illustrates a change in an expression level of IL-5, and FIG. 6E illustrates a change in an expression level of IL-13 in splenocytes of C57BL/6 mice injected with Con A and BE. (Mean ± SD from data (n = 3 per group); **p < 0·01 and ****p < 0·0001 vs. naïve control (NC) group; ##p < 0·01 and ####p < 0·0001 vs. con A group.);

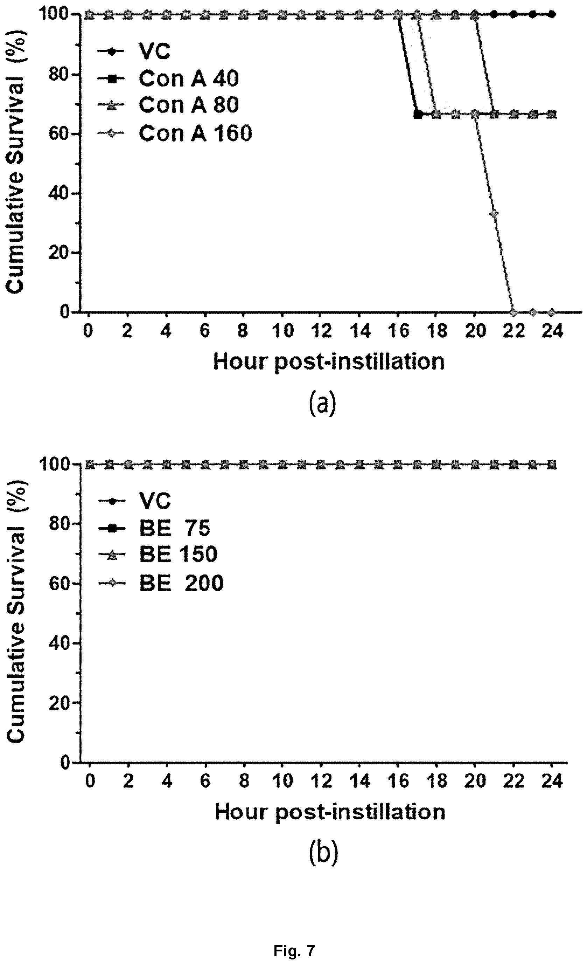

FIGS. 7A and 7B illustrate effects of Con A and BE injection on the survival rate of C57BL/6 mice in examples of the present disclosure, according to an example embodiment of the present disclosure, FIG. 7A illustrates hourly survival of up to 24 hours of VC (vehicle control) after injection of Con A at concentrations of 40, 80 or 160 mg/kg (body weight, BW), and FIG. 7B illustrates hourly survival of up to 24 hours of VC (vehicle control) after injection of BE at concentrations of 75, 160 and 200 mg/kg (body weight, BW);

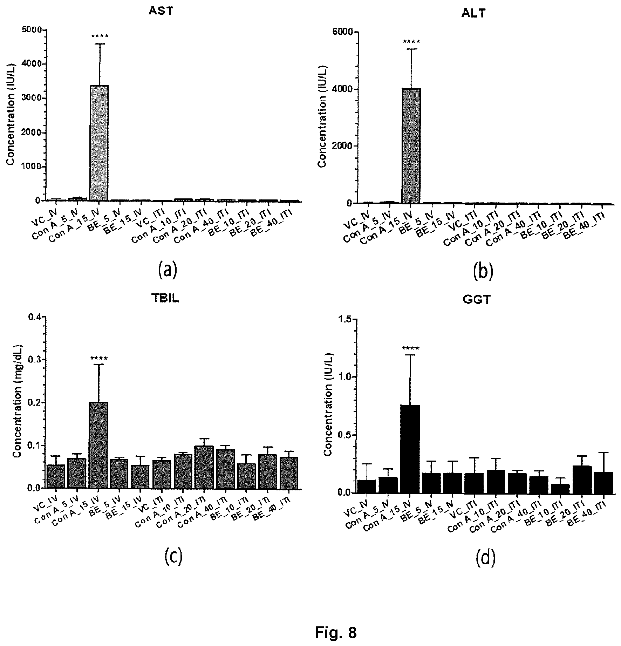

FIGS. 8A to 8D illustrate the results of changes in liver biomarker (AST, ALT, TBIL and GGT) levels in response to Con A and BE instillation in mice in examples of the present disclosure, according to an example embodiment of the present disclosure, in which biomarker concentrations were measured in the sera of mice administered Con A and BE (intravenous or intratracheal instillation, 5 to 15 or 10 to 40 mg/kg BW, respectively). (Mean ± SD (n = 5 mice per group), ****p < 0·0001 vs. VC, AST, aspartate aminotransferase; ALT, alanine aminotransferase; TBIL, total bilirubin level GGT, g-glutamyl transferase.);

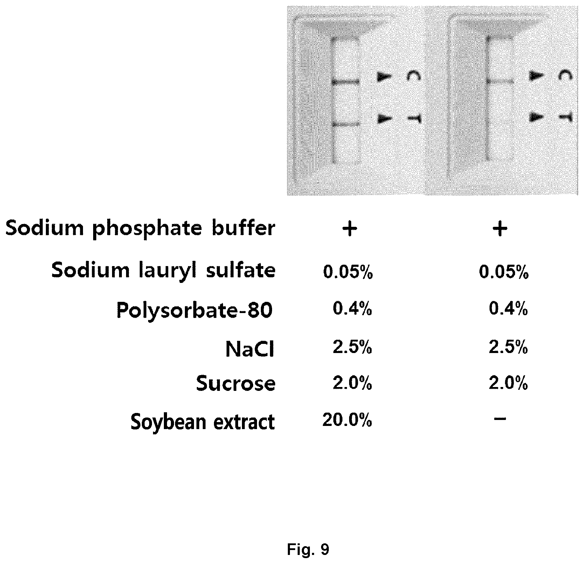

FIG. 9 illustrates an evaluation of the performance results of a COVID-19 antigen diagnostic test (RDT) performed using a soybean extract-containing specimen collection solution according to an example embodiment of the present disclosure in examples of the present disclosure, according to an example embodiment of the present disclosure;

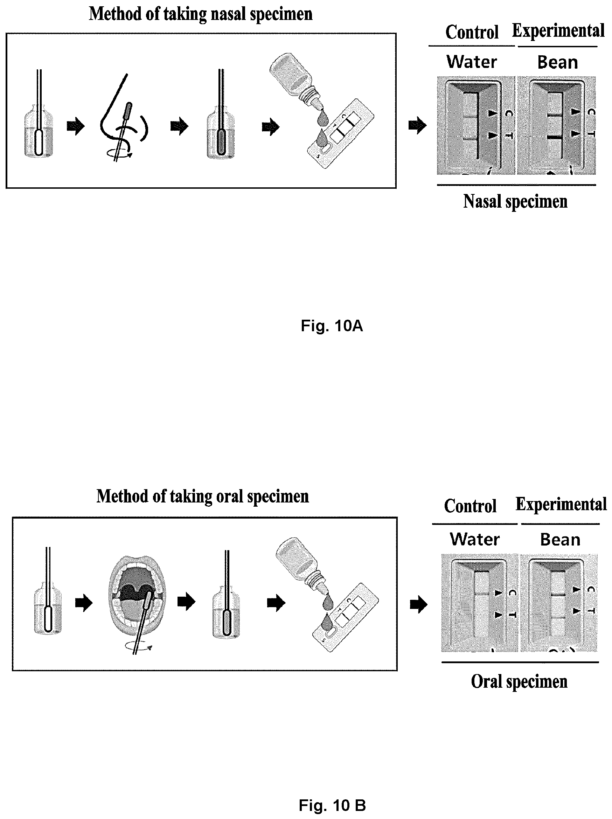

FIG. 10A illustrates an evaluation of COVID-19 specimen collection and antigen extraction performance of a soybean extract-containing specimen collection solution through the nasal specimen collection of patients with COVID-19 according to an example embodiment of the present disclosure in examples of the present disclosure, according to an example embodiment of the present disclosure;
FIG. 10B illustrates an evaluation of COVID-19 specimen collection and antigen extraction performance of a soybean extract-containing specimen collection solution through the oral specimen collection of patients with COVID-19 according to an example embodiment of the present disclosure in examples of the present disclosure, according to an example embodiment of the present disclosure; and

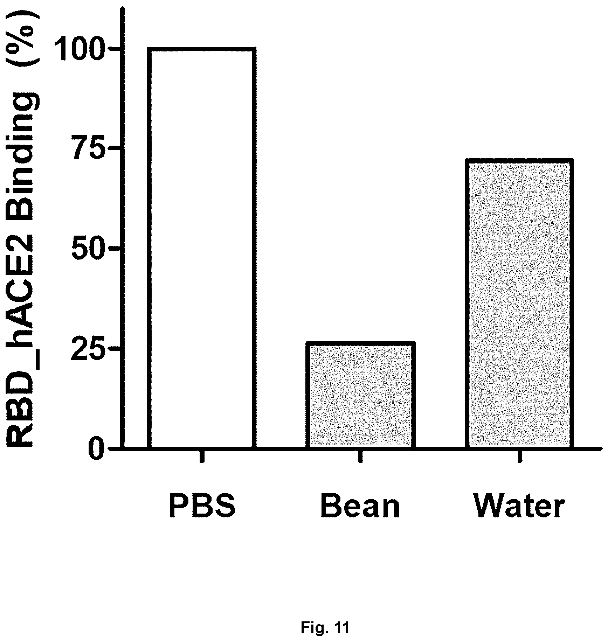

FIG. 11 illustrates an evaluation of a SARS-CoV-2 RBD spike protein neutralizing effect of a solution composition for collecting saliva specimens containing soybean extract according to an example embodiment of the present disclosure in examples of the present disclosure, according to an example embodiment of the present disclosure.

### DETAILED DESCRIPTION

Hereinafter, example embodiments of the present disclosure will be described in detail with reference to the accompanying drawings. In describing the present disclosure, when it is determined that the specific description of related known functions or configurations unnecessarily obscure the gist of the present disclosure, the detailed description therefor may be omitted. In addition, terms used herein are terms used to properly represent preferred example embodiments of the present disclosure. It may vary depending on the intent of users or operators, or custom in the art to which the present disclosure belongs. Accordingly, the definitions of these terms should be based on the contents throughout this specification. Like reference numerals in each drawing designate like elements.

Throughout this specification, the term "on" that is used to designate a position of one element with respect to another element includes both a case that the one element is adjacent to the another element and a case that any other element exists between these two elements.

Throughout this specification, when a part is referred to as "comprising" a component, it means that it may further include other components without excluding other components unless specifically described otherwise.

Hereinafter, the present disclosure will be described in more detail through example embodiments. However, these example embodiments are for illustrative purposes of the present disclosure, and the scope of the present disclosure is not limited to these example embodiments.

The present disclosure relates to a specimen collection solution, and according to an example embodiment of the present disclosure, the specimen collection solution may be used to collect a specimen used for a rapid antigen diagnostic test. According to an example embodiment of the present disclosure, the specimen collection solution may include soybean extract; sodium chloride; a surfactant; and an emulsifier.

According to an example embodiment of the present disclosure, the specimen collection solution may be used to obtain a specimen solution for a rapid antigen diagnosis, for example, directly used for specimen collection in the oral cavity (for example, saliva) and/or respiratory tract (for example, nasal cavity), and/or utilized for a pretreatment solution for obtaining a specimen solution. In other words, a specimen solution may be obtained by collecting a specimen in the oral cavity and/or in the respiratory tract (for example, nasal cavity) and treating (for example, mixing) the specimen with the specimen collection solution. It is possible to provide a specimen solution capable of improving the virus concentration, sensitivity, and accuracy suitable for a rapid antigen diagnostic test by increasing the desorption of viruses in the body (for example, saliva, secretions and/or tissues) and/or the substrate to which a specimen is attached (for example, a specimen collection tool) (for example, a cotton swab).

According to an example embodiment of the present disclosure, the specimen collection solution is collected by washing the oral cavity (for example, saliva) in the form of a gargle to capture intraoral viruses, and a virus (for example, infected or symptomatic) of a diagnosable concentration may be obtained. In some instances, the obtained specimen solution may be directly used for a rapid antigen diagnostic test without additional pretreatment (for example, treatment with a buffer solution for antigen extraction). In some instances, the sensitivity and accuracy of a test may be improved by increasing the activation of the antigen-antibody reaction in a rapid antigen diagnostic test. In some instances, it is possible to provide a one-step rapid antigen diagnostic method and a diagnostic kit for directly introducing the obtained specimen solution into a diagnostic device.

According to an example embodiment of the present disclosure, the specimen collection solution is absorbed and/or attached to a specimen collection tool for virus capture in the respiratory tract (for example, nasal cavity) to be used to collect a specimen in the respiratory tract (for example, nasal cavity). In addition, the specimen collection solution may be utilized as a pretreatment solution for collecting and diluting the virus in the specimen collection tool after the specimen collection act.

According to an example embodiment of the present disclosure, the specimen collection solution may be utilized as a pretreatment solution for collecting viruses from secretions (for example, saliva) taken from the human body.

According to an example embodiment of the present disclosure, the soybean extract is derived from soybean, which is a natural substance, and may include terminally truncated canavalin, which is a useful protein that is purified from a fermented soybean and has antiviral properties. The terminally truncated canavalin may prevent a virus from attaching to a receptor, detach the virus attached to the receptor, and/or convert the same into a natural state without infectivity. By using the interaction of the virus with the terminally truncated canavalin, the terminally truncated canavalin may be utilized as a composition for preventing and/or treating a viral infection, or a pretreatment composition for washing a virus and obtaining a specimen for a virus test.

According to an example of the present disclosure, the soybean extract may be included in an amount of 0.1 wt% 40 wt%; 0.5 wt% to 40 wt%; 1 wt% to 30 wt%; 5 wt% to 25 wt%; 0.1 wt% to 10 wt%; 0.5 wt% to 5 wt%; or 1 wt% to 3 wt% with respect to the specimen collection solution. When the soybean extract is included within the above range, it is possible to provide a specimen solution that may improve the sensitivity (for example, display intensity) and accuracy of a rapid antigen test by increasing the desorption of viruses attached to the human body, substrate (for example, specimen collection tool) (for example, cotton swab), etc. Moreover, it is possible to provide a specimen solution capable of collecting a specimen using a simple specimen collection method (for example, gargling) without additional pretreatment and directly introducing the same into a diagnostic device, as well as improving sensitivity (for example, display intensity).

According to an example of the present disclosure, the terminally truncated canavalin is C-terminally truncated canavalin, and may include a C-terminally truncated sequence among canavalin amino acid sequences. For example, as shown in FIG. 5, the C-terminally truncated sequence may be identified by LSSQDKPFNL.

According to an example of the present disclosure, the terminally truncated canavalin may interfere with and inhibit the binding of a receptor binding domain to a receptor of a virus, and may surround and desorb the already bound virus. In certain instances, it may provide a function of inhibiting binding of a viral spike receptor binding domain to a receptor. In certain instances, binding of the viral spike receptor binding domain to the receptor may be prevented or desorbed. In certain instances, the infectivity may be lowered by neutralizing the virus.

According to an example embodiment of the present disclosure, the terminally truncated canavalin may be obtained through a preparation process including: fermenting soybeans, soybean hulls or both; and isolating the terminally truncated canavalin from a fermentation product. For example, the preparation process may be carried out under fermentation conditions capable of removing a ConA toxic protein having toxicity to soybeans (for example, Canavalia gladiata) through a fermentation process and securing an antiviral useful protein.

According to an example of the present disclosure, a useful protein, terminally truncated canavalin, may be formed by proteolysis during the fermentation. For example, the fermentation may proceed with a fermentation process at 10°C to 45°C; 20°C to 40°C; room temperature to 40°C; or a temperature of 30°C to 40°C and under a dissolved oxygen atmosphere of 30% to 50% for at least 10 hours; at least 1 day; or 1 to 5 days; or 1 to 4 days. For example, the soybean is Canavalia gladiata, and soybeans other than Canavalia gladiata may be applied. For example, the fermentation may be performed at a pH of 5 to 8, a pH of 6 to 7.5, or preferably in a neutral region. For example, the solvent may include water, an organic solvent, or both, for example, the organic solvent may include at least one of an alcohol having 1 to 6 carbon atoms, butylene glycol, and propylene glycol.

According to an example of the present disclosure, the isolation of the terminally truncated canavalin from the fermentation product may include removing toxic substances from the fermentation product and isolating a useful protein sequence to obtain terminally truncated canavalin. For example, the terminally truncated canavalin may be isolated from the fermentation product using size exclusion chromatography.

According to an example embodiment of the present disclosure, the sodium chloride utilizes the principle of osmotic pressure to rupture the virus membrane to expose the antigen, thereby providing a specimen solution applicable to a diagnostic test without additional pretreatment for antigen extraction (for example, buffer treatment for antigen extraction). In certain instances, there is provided a specimen solution capable of exposing antigens by rupturing the viral membrane present in the oral cavity (for example, saliva), respiratory tract (for example, nasal cavity) and/or substrate, and directly introducing the specimen solution into a diagnostic device without additional pretreatment for antigen extraction (for example, buffer treatment for antigen extraction). In some instances, a rapid antigen diagnostic test may be realized by a one-step process applied immediately after collection of a specimen solution with a simple gargle without additional pretreatment for antigen extraction (for example, buffer treatment for antigen extraction).

According to an example of the present disclosure, in order to induce osmosis, sodium chloride (NaCI) may be included in an amount of 0.1 wt% to 5 wt%; 0.5 wt% to 3 wt%; or 1 wt% to 3 wt% with respect to the specimen collection solution. When the sodium chloride is included within the above range, a specimen may be collected using a simple specimen collection method (for example: gargling) without additional pretreatment, and further, the specimen may be directly introduced into a diagnostic device, and sensitivity (for example, display intensity) may be improved.

According to an example embodiment of the present disclosure, the surfactant may include an anionic surfactant for food additives, a cationic surfactant, or both. In certain instances, the surfactant may provide an environment for optimal antigen-antibody reaction in a strip of an antigen diagnostic device to which a lateral flow assay is applied, and may improve the sensitivity of a test. In certain instances, the improvement may be achieved by the simultaneous addition of a surfactant and an emulsifier. In certain instances, the surfactant is capable of activating functional and/or immunological structural binding of an antigen-antibody site.

According to an example of the present disclosure, the surfactant may include, for example, at least one of sodium lauryl sulfate, sodium myristyl sulfate, sodium N-lauroyl sarcosinate, sodium N-myristyl sarcosinate, monoglyceride sulfate, sodium lauroyl sarcosinate, lauric acid diethanolamide, polyoxyethylene, lauric acid diethanolamide, N-lauryldiamino ethyl glycine, N-myristyl diamino ethyl glycine, N-alkyl-N-carboxymethyl ammonium betaine, and sodium 2-alkyl-1-hydroxyethyl imidazoline betaine. In certain instances, it may be preferable to be selected from the sodium lauryl sulfate, sodium myristyl sulfate, and sodium N-lauroyl sarcosinate.

According to an example of the present disclosure, the surfactant may be included in an amount of 0.01 wt% to 2 wt%; 0.05 wt% to 1 wt%; or 0.1 wt% to 0.5 wt% with respect to the specimen collection solution. When the surfactant is included within the above range, the sensitivity and accuracy of a rapid antigen diagnostic test may be improved by activating functional or immunological structural binding of an antigen-antibody site.

According to an example embodiment of the present disclosure, the emulsifier prevents the antigen present in saliva from adsorbing to the surface on the strip, and minimizes aggregation between various proteins to optimize a specimen collection solution to be prepared in a form suitable for binding an antigen recognized by an antibody.

According to an example of the present disclosure, the emulsifier is an emulsifier for food additives, and may include, for example, at least one of polysorbate 80, polysorbate 60, polysorbate 20, sucrose fatty acid ester, glycerin fatty acid ester, propylene glycol fatty acid ester, sorbitan fatty acid ester, organic acid monoglyceride, and lecithin. In certain instances, it may be preferable to be selected from polysorbate 80, polysorbate 60, polysorbate 20.

According to an example of the present disclosure, the emulsifier may be included in an amount of 0.01 wt% to 2 wt%; 0.05 wt% to 1 wt%; or 0.1 wt% to 0.5 wt% with respect to the specimen collection solution. When the emulsifier is included within the above range, the sensitivity and accuracy of a rapid antigen diagnostic test may be improved by providing an environment for the optimal reaction of antigen-antibody.

According to an example of the present disclosure, the mixing ratio (mass ratio) of the surfactant to the emulsifier may be in a range of 0.01 : 1 to 1.5 : 1; 0.1 : 1 to 1.2 : 1; 0.8 : 1 to 1.2 : 1; 0.9 : 1 to 1 : 1; about 1 : 1; 0.1 : 1 to 1 : 1; or 0.11 : 1 to 0.25 : 1. In other words, in order to provide an optimized specimen collection solution for improving the sensitivity of rapid antigen test diagnosis, the surfactant may have a low content or may be included in substantially the same mass.

According to an example embodiment of the present disclosure, the specimen collection solution includes a solvent, and the solvent includes water, an organic solvent, or both, for example, the organic solvent may include at least one of an alcohol having 1 to 6 carbon atoms, butylene glycol, and propylene glycol, preferably water. The water may be pure water, tertiary distilled water, and sterile distilled water applied to specimen treatment of a rapid antigen diagnostic test, but is not limited thereto. According to an example of the present disclosure, the solvent may be included in an amount of a balance or 1 wt% to 99 wt%; 2 wt% to 99 wt%; 5 wt% to 95 wt%; or 10 wt% to 90 wt% with respect to the specimen collection solution.

According to an example embodiment of the present disclosure, the specimen collection solution may be used for a rapid antigen diagnostic test for enveloped viruses, non-enveloped viruses, or both. In certain instances, the specimen collection solution may be used for a diagnostic test of respiratory viral infections.

For example, the respiratory virus may include at least one of influenza virus (for example, influenza virus A (IVA) and influenza virus B (IVB)), coronavirus (for example, COVID-19), syncytial virus (RSV, such as respiratory syncytial virus A (RSVA) and respiratory syncytial virus B (RSVB)), adenovirus, human bocavirus, rhinovirus, human metapneumovirus (hMPV), Middle East Respiratory Virus (MERS-CoV), SARS-associated coronavirus (SARS-coV), human parainfluenza virus 1 (HPIV 1), human parainfluenza virus 2 (HPIV 2), and human parainfluenza virus 3 (HPIV 3).

In certain instances, the specimen collection solution may be used for a diagnostic test for coronavirus and/or coronavirus infection (COVID-19).

According to an example embodiment of the present disclosure, the specimen collection solution may be used for taking an oral and/or respiratory (for example, nasal cavity) specimen, for example, for collecting a specimen for a rapid antigen diagnostic test.

According to another example embodiment of the present disclosure, the specimen collection solution may be further used to obtain a specimen solution for testing by collecting and capturing enveloped viruses, non-enveloped viruses, or both attached to and/or included in body fluid, cells (ex *vivo* and/or *in vivo*), a substrate for taking a specimen, human tissues (for example, oral cavity; respiratory tract such as bronchus, trachea, nose, and the like), and the like. For example, a specimen solution may be obtained by collecting viruses included in or attached to at least one of an oral cavity surface, a nasal cavity surface, and runny nose. In certain instances, the specimen solution obtained may be directly applied to a rapid antigen diagnostic test. For example, a one-step diagnostic test may be realized by directly applying a specimen solution obtained by gargling in the oral cavity to the rapid antigen diagnostic test.

The present disclosure may provide a diagnostic kit for a rapid antigen diagnostic test including the specimen collection solution according to the present disclosure. According to an example embodiment of the present disclosure, the diagnostic kit may include the specimen collection solution and the rapid antigen diagnostic device according to the present disclosure. The rapid antigen diagnostic device may be appropriately selected according to a test target, and may include a rapid antigen diagnostic device known in the technical field of the present disclosure.

According to an example of the present disclosure, in the rapid antigen diagnostic device, a specimen solution instilled from a sample inlet (for example, a specimen instillation site) may be absorbed and flow through a capillary phenomenon, and may form a strip capable of identifying a display for diagnosis in a reaction region (for example, an indicator antibody region) according to a test target. In addition, if necessary, the rapid antigen diagnostic device may further include a configuration known in the technical field of the present disclosure, which is not specifically mentioned in this document. For example, a test may be performed by instilling a specimen solution obtained after gargling with the specimen collection solution for about 2 minutes to a specimen instillation site, and the display by an antigen-antibody reaction (for example, color band generation) may be identified.

According to an example embodiment of the present disclosure, the diagnostic kit may be for detecting enveloped viruses, non-enveloped viruses or both and/or diagnosing related disorders and/or diseases. In certain instances, the diagnostic kit may be for diagnosing respiratory viruses, for example, for diagnosing COVID-19.

According to an example embodiment of the present disclosure, the diagnostic kit may take a specimen from the oral cavity and/or respiratory tract (for example, nasal cavity). As an example of the present disclosure, a one-step rapid antigen diagnostic test in which a specimen solution is obtained by gargling a specimen collection solution according to the present disclosure in the oral cavity and the specimen solution is directly introduced into a diagnostic kit may be performed. This may reduce the stages of the rapid antigen diagnostic test, and improve the sensitivity and accuracy.

As an example of the present disclosure, the specimen collection solution according to the present disclosure may be adsorbed or absorbed on a substrate for specimen collection (for example, a specimen collection tool) (for example, a cotton swab), and using the same, a specimen may be taken in the oral cavity and/or nasal cavity. After treating the substrate containing the specimen with the specimen collection solution according to the present disclosure (for example, mixing in the solution), the solution may be introduced into a diagnostic device. It is possible to provide a specimen solution capable of improving sensitivity and accuracy by increasing desorbing of the target virus within the infected nasal cavity.

As an example of the present disclosure, after taking a specimen from the oral cavity and/or nasal cavity with a substrate, the specimen may be pre-treated (for example, mixed) with the specimen collection solution according to the present disclosure to obtain a specimen solution, and may be applied to a rapid antigen diagnostic test. It is possible to provide a specimen solution capable of improving sensitivity and accuracy by increasing desorbing of the virus attached to the substrate.

According to an example embodiment of the present disclosure, the diagnostic kit may be free of a buffer solution for antibody extraction. In other words, the diagnostic kit may conduct a test by directly introducing the specimen solution obtained as the specimen collection solution according to the present disclosure to a specimen instillation site. Since it is possible to omit a treatment process of a buffer for antibody extraction during a specimen pretreatment process, the stages of the diagnostic test process may be minimized and the test sensitivity and accuracy by the specimen collection solution may be improved.

An aspect of the present disclosure provides a method for a rapid antigen diagnostic test using the specimen collection solution according to the present disclosure.

According to an example embodiment of the present disclosure, the method may provide a one-step rapid antigen diagnostic test method including: collecting a specimen solution; and injecting the specimen solution into an antigen diagnostic device and performing a diagnosis.

According to an example embodiment of the present disclosure, the collection of the specimen solution may include oral gargling of the specimen collection solution according to the present disclosure to collect the specimen solution (for example, a saliva specimen solution). In an example of the present disclosure, in the gargling, the gargling may be performing for 10 seconds or longer; 30 seconds or longer; 1 minute or longer; 2 minutes or longer; 2 to 10 minutes; or 2 to 5 minutes. In addition, in another example, the specimen solution may be collected by contacting the specimen collection solution with a substrate, cell, tissue, etc. that contains or attaches to saliva. This may be a washing process for specimen solution collection, and may be performed *ex vivo* and/or *in vivo.*

According to an example embodiment of the present disclosure, the injection of the specimen solution into the antigen diagnostic device and the performance of the diagnosis may include directly injecting the specimen collected as the specimen collection solution to a specimen instillation site of a diagnostic kit. After the injection, a diagnostic test process may be performed according to the operation principle and display method of a diagnostic kit. For example, in a strip of a diagnostic kit, it may move and react according to the capillary principle to form a display (for example, color development or color band formation) that may identify a test target. It may be discriminated as negative or positive according to the occurrence of the display.

According to another example embodiment of the present disclosure, there is provided a rapid antigen test method including: collecting a specimen solution; and injecting the specimen solution into an antigen diagnostic device and performing a diagnosis.

As an example of the present disclosure, the collection of the specimen solution may include: adsorbing and/or absorbing the specimen collection solution according to the present disclosure on a substrate used for specimen collection (for example, a specimen collection tool) (for example, a cotton swab) and taking a specimen from the substrate at a specimen taking site (for example, oral cavity and/or nasal cavity); treating the substrate to which the specimen attached to the specimen collection solution according to the present disclosure to obtain a specimen solution; and introducing the specimen solution into a rapid antigen diagnostic device. In other words, it is possible to provide a specimen solution capable of improving sensitivity by increasing desorbing of viruses by the specimen collection solution according to the present disclosure.

According to an example embodiment of the present disclosure, in the injection of the specimen solution into the antigen diagnostic device and the performance of the diagnosis, the specimen solution may be free of a buffer solution for antigen extraction.

According to an example embodiment of the present disclosure, the method may not include a process of treating (for example, mixing) a buffer solution for antigen extraction in the specimen solution, and may provide a one-step process that is directly injected into the antigen diagnostic device immediately after the collection of the specimen solution.

In other words, in FIG. 1A, in the conventional *in vitro* diagnostic device, it is essential to use a specimen collection tool (for example, a cotton swab) or to collect saliva in a specimen container and then react with a buffer for antigen extraction. In the case of using the specimen collection solution (for example, a saliva specimen collection solution) according to the present disclosure as shown in FIG. 1B, it is possible to provide a method in which after gargling the oral cavity for 2 minutes, the gargle solution is directly instilled into a diagnostic device without antigen extraction, and then it is determined whether the solution is tested positive. This may minimize the pretreatment stages of the specimen and improve the sensitivity of the test.

As used herein, the term "gargle" may refer to oral or nasal washing for collecting saliva and viruses attached to the human oral mucosa. Moreover, in another example, the specimen collection solution may correspond to a pretreatment of a diagnostic test in which a virus attached to and/or containing a human mucous membrane, body fluid, etc. in addition to oral mucosa is washed for collection and a related solution is collected. The body fluid may mean a liquid form that flows out of the human body, such as saliva, runny nose, and urine.

Hereinafter, the present disclosure will be described in more detail by way of example embodiments. However, these example embodiments are for illustrative purposes of the present disclosure, and the scope of the present disclosure is not limited to these example embodiments.

### Example 1: Optimization of Solution Composition for Oral Specimen Collection

A COVID-19 antigen diagnostic test was performed to establish optimization conditions for a solution composition for oral specimen collection. In order to minimize the stages of specimen pretreatment, a method of exposing antigens was devised by bursting the virus membrane present in saliva utilizing the principle of osmotic pressure. As a composition to induce an osmotic phenomenon, a solution composition (aqueous solution) having sodium chloride (NaCI) concentrations of 1%, 2%, and 3%, respectively, was prepared.

As a control group for performance evaluation, an antigen elution buffer enclosed with an antigen diagnostic device was used, and heat inactivated SARS-CoV-2 (0810589CFHI, ZeptoMetrix, NY, USA) was used as an antigen.

As shown in FIG. 2, the composition in which each of 0.1% sodium lauryl sulfate and 0.1% polysorbate 80 was combined in the composition having a sodium chloride (NaCI) concentration of 1%, 2%, and 3% did not show an antigen-antibody-bound test-line band of an antigen diagnostic device (Device #2), and showed a test-line band of the composition added with each of 1% and 2% sodium chloride (Devices #3 and #4), but showed a weaker band intensity compared to a control (Device #1). The composition in which sodium chloride, 0.1% sodium lauryl sulfate and polysorbate 80 were combined (Device #5 to #7) showed similar band intensity to that of the control (Device #1). In particular, in the composition in which 1% sodium chloride, 0.1% sodium lauryl sulfate, and 0.1% polysorbate 80 were combined, the band was identified the fastest within 2 minutes and the band intensity was also the best. Thus, it was identified that the specimen collection solution to minimize the oral specimen pretreatment stage could be optimized in 1% sodium chloride, 0.1% sodium lauryl sulfate and 0.1% polysorbate 80 (described as a one-step gargle in FIG. 2).

### Preparation Example: Soybean Extract (Bean, BE): TCan (truncated canavalin)

### (1) Preparation and Purification of TCan (truncated canavalin)

Proteolysis was conducted on sword beans (Canavalia gladiata) by Bacillus subtilus under dissolved oxygen (20% to 40% saturation) and pH 7.0 conditions and 33°C (or in purified water (96.059% to less than 100%)) for 4 days. After proteolysis, the product was centrifuged (12,000xg) at 4°C for 15 minutes, and the supernatant was collected and then lyophilized (hereinafter, BE (fermented soybean extract)). Final purification was performed by size exclusion chromatography (SEC) using a WTC-050S5 column (Wyatt Technology, Santa Barbara, CA) equilibrated with a PBS buffer solution (140 mM NaCl, 2.7 mM KCI, 6.5 mM Na₂H₂PO, 1.5 mM KH₂PO, pH 7.4). The pure protein was frozen in LN2 stored at -80°C, and the SEC profile of TCan is shown in FIG. 3.

### (2) Analysis of Total Mass Value of Protein using UPLC-ESI-QTOF Mass Spectrometer (Top-Down Method)

On-line top-down MS was performed on a waters ACQUITY UPLC I-Class system connected to a Synapt G2-S QTOF mass spectrometer (Waters Corp., USA). 2 µl of purified TCan was injected onto a 2.1 x 50 mm ACQUITY UPLC BEH300 C4 column (particle size of 1.7 µm). The concentration gradient started at 5% B (0.1% formic acid in acetonitrile) and delivered at 400 µl rising to 95 % B at 6.7 minutes and 5% B at 9.0 minutes.

The mass spectrometer was operated in an ESI-Positive MSE continuum data acquisition mode, a capillary voltage of 3.0 kV, a cone voltage of 50 V, desolvation gas of 800 L/hr, a source temperature of 120°C, and a desolvation temperature of 300°C. Data was collected at 1 spectra/second at 500 to 4,000 m/z. The maximum entropy algorithm (MaxEnt1) was used for mass deconvolution. The deconvolution was performed in the range of 1,500 to 2,600 Da, centered on the expected mass and target resolution of 0.5 Da, by repeating MaxEnt1 100 times. The electrospray spectrum of TCan is shown in FIG. 4.

### (3) N-terminal Sequencing of Tcan

Protein samples were transferred from an SDS-PAGE gel to PVDF membranes and protein bands transferred from the membrane were analyzed using an LC 492 Protein Sequencing System (Applied Biosystems Instruments, USA). The results are shown in FIG. 5.

### Biotoxicity Test

### (1) Mitogenic Response by Con A and BE (Fermented Soybean Extract) in Rat Splenocytes

Con A is well known to induce mitogenic responses in splenocytes that activate the immune system, recruit lymphocytes and induce cytokine production. To compare the mitogenic effects induced by Con A and BE, T helper (Th) cytokines produced by treating splenocytes with Con A or BE at a concentration of 2·5 µg/mL ((IFN-g for Th1, IL-17 for Th17, and IL-4, IL-5, and IL-13 for Th2 cells) were investigated. The results are shown in FIGS. 6A to 6E. In comparison with BE in FIGS. 6A to 6E, it showed that Con A induced a much higher production of all Th cytokines. The production of IFN-g, IL-17, IL-5 and IL-13 increased in BE-treated splenocytes during the experimental period, but the production was significantly lower than that of the Con A treatment group.

### (2) Effects of Con A and BE on Survival Rate of Mice

To investigate and compare the toxic effects of Con A and BE in an iving system, the effect of Con A and BE on mortality after intratracheal instillation in C57BL/6 mice was investigated. The results are shown in FIGS. 7A and 7B. In FIGS. 7A and 7B, the survival rate was monitored hourly in C57BL/6 mice divided into 7 groups: a vehicle control group (VC); Groups treated with Con A (Con A 40, Con A 80, Con A 160 groups) at concentrations of 40, 80 and 160 mg/kg body weight (BW) and groups treated with BW at concentrations of 75, 160 and 200 mg/kg BW (BE 75, BE 160, BE 200 groups) (treatment time: 24 hours). The VC and BE treatment groups showed a survival rate of 100%, whereas the Con A treatment group showed a decreased survival rate. The survival rate of the mice in the Con A treatment group started to decrease from 17 hours after the treatment, and the survival rate of the mice in the Con A 160 treatment group was not observed until 24 hours after the treatment. In the mice of the Con A 40 and Con A 80 groups, 60% survival was observed after 24 hours of treatment. These results indicate that BE is not toxic to mice, which is consistent with the *in vitro* cytotoxicity results.

### (3) Effects of Con A and BE on Liver Toxicity in Rats

Con A activates T cells to secrete cytokines that cause liver damage. To compare the effects of Con A and BE on liver damage, the values of serum AST, ALT, TBIL and GGT, biomarkers of liver function, were evaluated 24 hours after Con A and BE administration to mice. The results are shown in FIGS. 8A to 8D. FIGS. 8A to 8D show that the mode of administration affects liver biomarker levels. In both Con A and BE-treated mice, intratracheal instillation had no significant effect on the level of liver biomarkers, but it may be seen that a high concentration of Con A (15 mg/kg BW) administered intravenously significantly increased the expression of assorted biomarkers (p value < 0.05).

### Example 2: Optimization of Solution Composition for Collecting Saliva Specimens Containing Soybean Extract

A COVID-19 antigen diagnostic test was performed to establish optimization conditions for a solution composition for collecting saliva specimens including soybean extract. For the soybean extract, a fermentation extract filtrate using fermented bacteria was used. Sodium phosphate buffer (MgCl₂, KCI, sodium benzoate, pH 7.5) was used as a buffer solution for antigen extraction, and 2.5% of sodium chloride (NaCI) was added as a composition to induce an osmotic phenomenon. To supplement the salty taste of sodium chloride, 2% of sugar (sucrose) was added, and a composition (aqueous solution) was prepared with 0.05% of sodium lauryl sulfate and 0.4% of polysorbate-80. As a control group for performance evaluation, sterile distilled water was used as a composition instead of 20% soybean extract, and heat inactivated SARS-CoV-2 (0810589CFHI, ZeptoMetrix, NY, USA) was used as an antigen.

As shown in FIG. 9, the composition combining soybean extract showed high band intensity of an antigen-antibody-bound test-line band of an antigen diagnostic device. On the other hand, the band intensity of the test-line band of the control group including sterile distilled water was shown to be low. As a result, it was identified that as an antigen extraction solution to minimize the saliva specimen pretreatment stage, it was optimized with 20% soybean extract containing 2.5% sodium chloride, 2% sugar (sucrose), 0.05% sodium lauryl sulfate and 0.4% polysorbate 80.

### Example 3: Specimen Taking Effect of Solution Composition for Specimen Collection Containing Soybean Extract

After soaking a cotton swab with a specimen collection solution containing soybean extract (Bean) or sterile distilled water (Water), SARS-CoV-2 was applied to the nasal cavity and oral cavity to evaluate the specimen taking ability. As the actual specimen, the present inventor's specimen was utilized as the present inventor confirmed COVID-19 omicron. Specimens were taken by using a cotton swab soaked in a specimen collection solution containing soybean extract (Bean) or sterile distilled water (Water) for each nasal cavity and oral cavity. The cotton swab that came into contact with the specimen was immersed in the specimen collection solution again to wash the cotton swab, and then the cotton swab was removed. The band intensity of the test-line band was checked by directly instilling the specimen collection solution into an antigen diagnostic device.

As shown in FIG. 10A, according to the method of taking a nasal specimen, it is possible to identify the high band intensity of the test-line band of the antigen diagnostic device in a composition for collecting specimens containing soybean extract compared to the sterile distilled water (Water) as a control.

As shown in FIG. 10B, a very weak band was identified in the test-line band of the antigen diagnostic device of the composition for collecting specimens containing sterile distilled water (Water) as a control according to the method of taking an oral specimen, whereas the composition for collecting specimens containing soybean extract (Bean) was identified to show a high-intensity test-line band. From this result, it was determined that the soybean extract (Bean) was easy to take specimens due to its binding force with the virus and had an excellent taking effect at a high concentration. According, it was attempted to prove the binding force of the saliva specimen collection solution composition containing soybean extract to SARS-CoV-2 RBD spike protein through Example 4.

### Example 4: Neutralizing Effect of SARS-CoV-2 RBD Spike Protein of Solution Composition for Collecting Saliva Specimens Containing Soybean Extract

The virus capturing efficacy analysis of the solution composition for collecting saliva specimens containing soybean extract (Bean) and the solution composition for collecting saliva specimens containing sterile distilled water (Water) was performed. The neutralizing effect of SARS-CoV-2 virus was performed according to the manufacturer's manual of the COVID-19 Neutralizing Antibody ELISA Kit (Abnova, Cat. KA6111). After taking a solution composition for collecting saliva specimens containing soybean extract (Bean) and sterile distilled water (Water), the solution composition was neutralized with SARS-CoV-2 spike RBD Rabbit Fc-Tag protein provided in the COVID-19 Neutralizing Antibody ELISA Kit at 37°C for 2 hours. The solution composition for collecting saliva specimens containing the neutralized soybean extract (Bean) and sterile distilled water (Water) and SARS-CoV-2 spike RBD Rabbit Fc-Tag protein were added to the hACE2-coated ELISA plate, and the reaction was performed with hACE2 at 37°C for 2 hours. After washing 4 times with the washing buffer provided in the kit, HRP-conjugated Goat Anti-Rabbit Antibody was added and reacted at 37°C for 1 hour. After washing 4 times with washing buffer, TMB reagent was added and reacted for 5 minutes, stop solution (1N HCI) was added, and absorbance at 450 nm was measured. As a negative control used in the neutralization reaction, PBS was used instead of the sample. The neutralizing effect was calculated as a percentage by relative comparison of the neutralizing force of the sample compared to the negative control.

As shown in FIG. 11, when the binding force of the control hACE2 and SARS-CoV-2 spike RBD Rabbit Fc-Tag protein was 100%, it was identified that the solution composition for collecting saliva specimens containing soybean extract (Bean) was neutralized with spike RBD Rabbit Fc-Tag protein and inhibited binding to hACE by 73%, and that the neutralizing force with SARS-CoV-2 spike RBD Rabbit Fc-Tag protein was excellent. However, the solution composition for collecting saliva specimens containing sterile distilled water (Water) inhibited binding force to hACE by 28%, and neutralizing force of SARS-CoV-2 spike RBD Rabbit Fc-Tag protein was weak.

The specimen collection solution according to the present disclosure may be applied to a test performed by an *in vitro* diagnostic device (IVD) used for a rapid diagnosis of COVID-19, and may improve sensitivity as well as minimize pretreatment stages. In other words, the specimen collection solution may provide a specimen collection solution (for example, a saliva specimen collection solution) for minimizing specimen pretreatment stages of a saliva-based COVID-19 antigen diagnostic test and a specimen collection method using the same. In addition, the specimen collection solution according to the present disclosure may provide a specimen solution that may improve the sensitivity, speed, and accuracy of the COVID-19 antigen diagnostic test, which may be effectively applied to the oral cavity and/or nasal cavity.

Although the example embodiments have been described based on the limited example embodiments and drawings as described above, those skilled in the pertinent technical field may apply various technical modifications and variations from the foregoing descriptions. For example, even when the described technologies are performed in a different order from that in the described method, and/or the described components are coupled or combined in a manner different from that as described above, or are replaced or substituted with other components or equivalents, appropriate results may be achieved. Therefore, other implementations, other example embodiments, and equivalents to claims also fall within the scope of the following claims.

## Claims

1. A specimen collection solution comprising:
soybean-derived terminally truncated canavalin;
sodium chloride;
a surfactant; and
an emulsifier.

2. The specimen collection solution of claim 1, wherein the specimen collection solution comprises:
0.1 wt% to 40 wt% of soybean-derived terminally truncated canavalin;
0.1% wt% to 5 wt% of sodium chloride;
0.01 wt% to 2 wt% of a surfactant; and
0.01 wt% to 2 wt% of an emulsifier.

3. The specimen collection solution of claim 1, wherein the soybean-derived terminally truncated canavalin is C-terminally truncated canavalin.

4. The specimen collection solution of claim 1, wherein:
the soybean-derived terminally truncated canavalin comprises a C-terminally truncated sequence among canavalin amino acid sequences; and
the terminally truncated canavalin is derived from Canavalia gladiata or a fermented soybean.

5. The specimen collection solution of claim 1, wherein the soybean-derived terminally truncated canavalin is extracted from a fermentation product obtained by inoculating Bacillus subtilus into soybeans, soybean hulls or both and then fermenting the same at 30°C to 40°C.

6. The specimen collection solution of claim 1, wherein:
the surfactant comprises an anionic surfactant, a cationic surfactant, or both; and
the surfactant comprises at least one selected from the group consisting of sodium lauryl sulfate, sodium myristyl sulfate, sodium N-lauroyl sarcosinate, sodium N-myristyl sarcosinate, monoglyceride sulfate, sodium lauroyl sarcosinate, lauric acid diethanolamide, polyoxyethylene, lauric acid diethanolamide, N-lauryldiamino ethyl glycine, N-myristyl diamino ethyl glycine, N-alkyl-N-carboxymethyl ammonium betaine, and sodium 2-alkyl-1-hydroxyethyl imidazoline betaine.

7. The specimen collection solution of claim 1, wherein the emulsifier comprises at least one selected from the group consisting of polysorbate 80, polysorbate 60, polysorbate 20, sucrose fatty acid ester, glycerin fatty acid ester, propylene glycol fatty acid ester, sorbitan fatty acid ester, organic acid monoglyceride, and lecithin.

8. The specimen collection solution of claim 1, wherein a mixing ratio of the surfactant to the emulsifier is in a range of 0.01 : 1 to 1.5 : 1 (w/w).

9. The specimen collection solution of claim 1, wherein the specimen collection is collected from an oral cavity or nasal cavity.

10. The specimen collection solution of claim 1, wherein the solution is a gargle solution.

11. A diagnostic kit for a one-step rapid antigen diagnostic test, the diagnostic kit comprising:
the specimen collection solution of claim 1; and
a rapid antigen diagnostic device.

12. The diagnostic kit of claim 11, wherein the diagnostic kit is for diagnosing respiratory viruses.
